(19) Europäisches Patentamt
European Patent Office
Office européen des brevets

(11) Veröffentlichungsnummer: **0 176 050**
**B1**

(12) # EUROPÄISCHE PATENTSCHRIFT

(45) Veröffentlichungstag der Patentschrift: 25.01.89

(21) Anmeldenummer: 85111939.6

(22) Anmeldetag: 20.09.85

(51) Int. Cl.⁴: **C 07 F 9/09**, C 07 F 9/24, C 07 F 9/65, C 07 F 9/15, A 61 K 31/66

ERRATUM

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | LAUTET BERICHTIGT:<br>SHOULD READ :<br>DEVRAIT ETRE LU : |
|---|---|---|---|
| 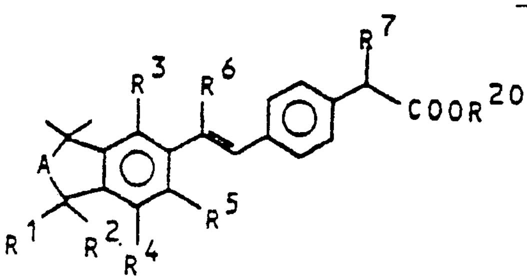 | 4 | 53 | 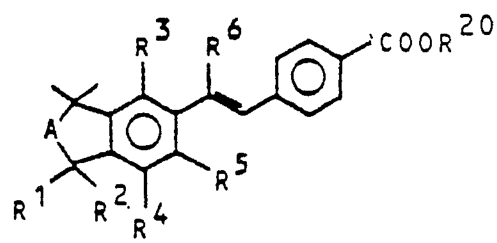 |
| A,$R^1$,$R^2$,$R^3$,$R^4$,$R^5$,$R^6$ und $R^7$ | 4 | 62 | A,$R^1$,$R^2$,$R^3$,$R^4$,$R^5$ und $R^6$ |

Tag der Entscheidung über die Berichtigung / Date of decision on rectification: 13.03.89

Ausgabe- und Veröffentlichungstag: / Issue and publication date: 03.05.89

Patbl.Nr / EPB no: 89/18

(19) Europäisches Patentamt
European Patent Office
Office européen des brevets

(11) Veröffentlichungsnummer: **0 176 050**
**B1**

(12) **EUROPÄISCHE PATENTSCHRIFT**

(45) Veröffentlichungstag der Patentschrift: **25.01.89**

(51) Int. Cl.⁴: **C 07 F 9/09**, C 07 F 9/24, C 07 F 9/65, C 07 F 9/15, A 61 K 31/66

(21) Anmeldenummer: **85111939.6**

(22) Anmeldetag: **20.09.85**

(54) **Phosphorsäurevinylbenzylester, ihre Herstellung und Verwendung.**

(30) Priorität: **22.09.84 DE 3434945**
**19.10.84 DE 3438386**

(43) Veröffentlichungstag der Anmeldung:
**02.04.86 Patentblatt 86/14**

(45) Bekanntmachung des Hinweises auf die Patenterteilung:
**25.01.89 Patentblatt 89/4**

(84) Benannte Vertragsstaaten:
**AT BE CH DE FR GB IT LI NL SE**

(56) Entgegenhaltungen:
**DE-A-2 854 354**
**DE-A-3 202 118**

(73) Patentinhaber: **BASF Aktiengesellschaft, Carl-Bosch- Strasse 38, D-6700 Ludwigshafen (DE)**

(72) Erfinder: **Wuest, Hans- Heiner, Dr., Unteres Bieth 10, D-6901 Dossenheim (DE)**
Erfinder: **Frickel, Fritz- Frieder, Dr., Silvanerweg 7, D-6705 Deidesheim (DE)**
Erfinder: **Nuerrenbach, Axel, Dr., Koenigsberger Strasse 7, D-6718 Gruenstadt (DE)**

**EP 0 176 050 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Phosphorsäurevinylbenzylester, sowie Verfahren zu deren Herstellung.

Es ist bekannt (DE-OS-2 854 354, DE-OS-3 202 118), daß Stilben-Derivate pharmakologische Wirkungen bei der topischen und systemischen Therapie von Neoplasien, Akne, Psoriasis und anderen dermatologischen Affektionen aufweisen.

Es wurde nun gefunden, daß Phosphorsäurevinylbenzylester der Formel I

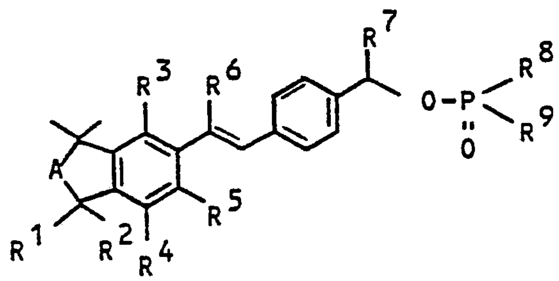

I,

worin

A einen gegebenenfalls durch $C_{1-4}$-Alkylgruppen substituierten Methylen- oder Ethylenrest,
$R^1$ und $R^2$ Wasserstoffatomen oder Methylgruppen
$R^3$ ein Wasserstoffatom, eine Methyl-, Hydroxy- oder $C_{1-6}$-Alkoxygruppe
$R^4$ und $R^5$ Wasserstoffatome, $C_{1-4}$-Alkyl- oder Methoxygruppen oder Halogenatome
$R^6$ ein Wasserstoffatom, eine $C_{1-6}$-Alkyl- oder $C_{3-6}$-Cycloalkylgruppe
$R^7$ ein Wasserstoffatom oder eine $C_{1-4}$-Alkylgruppe und
$R^8$ und $R^9$ die Reste $-OR^{10}$ oder $-NR^{11}R^{12}$ bedeuten, worin
$R^{10}$ ein Wasserstoffatom oder eine $C_{1-8}$-Alkyl-, Phenyl- oder Benzylgruppe,
$R^{11}$ und $R^{12}$ Wasserstoffatome, $C_{1-6}$-Alkylgruppen oder zusammen mit dem Stickstoffatom, an das sie gebunden sind, einen Pyrrolidino- Piperidino- oder Morpholino-Rest bedeuten und wobei jeweils zwei Reste
$R^{10}$, $R^{11}$ und/oder $R^{12}$ zusammen auch für eine Ethylen - oder Propylengruppe stehen können,

sowie deren physiologisch verträgliche Salze, ein besseres Wirkungsspektrum besitzen.

Bevorzugt sind die Verbindungen, in denen
A einen gegebenenfalls durch eine Methylgruppe substituierten Methylen- oder Ethylenrest darstellen,
$R^1$ und $R^2$ die angegebene Bedeutung besitzen,
$R^3$, $R^4$ und $R^5$ Wasserstoffatome,
$R^6$ ein Wasserstoffatom oder eine Methylgruppe,
$R^7$ Wasserstoff und
$R^8$ und $R^9$ eine Hydroxy- oder $C_{1-4}$-Alkoxygruppe bedeuten.

Typische Beispiele für erfindungsgemäße Verbindungen sind:

Phosphorsäure-4-[2-(5,6,7,8-tetrahydro-5,5,8,8-tetramethyl-2-naphthyl)-1-propenyl]benzylester
Phosphorsäure-4-[2-(2,3-dihydro-1,1,3,3-tetramethyl-5(1H)-indenyl)-1-propenyl]benzylester
Phosphorsäure-4-[2-(2,3-dihydro-1,1,2,3,3-pentamethyl-5(1H)-indenyl)-1-propenyl]benzylester
Phosphorsäure-4-[2-(5,6,7,8-tetrahydro-3,5,5,8,8-pentamethyl-2-naphthyl)-1-propenyl]benzylester
Phosphorsäure-4-[2-(5,6,7,8-tetrahydro-3-methoxy-5,5,8,8-tetramethyl-2-naphthyl)-1-propenyl]benzylester
Phosphorsäure-4-[2-(5,6,7,8-tetrahydro-1,3-dimethoxy-5,5,8,8-tetramethyl-2-naphthyl)-1-propenyl]benzylester
Phosphorsaure-4-[2-(5,6,7,8-tetrahydro-1,4-dimethoxy-5,5,8,8-tetramethyl-2-naphthyl)-1-propenyl]benzylester
Phosphorsäure-4-[2-(3-ethyl-5,6,7,8-tetrahydro-5,5,8,8-tetramethyl-2-naphthyl)-1-propenyl]benzylester
Phosphorsäure-4-[2-(5,6,7,8-tetrahydro-5,5,8,8-tetramethyl-3-(2-methylpropyl)-2-naphthyl)-1-propenyl]benzylester
Phosphorsäure-4-[2-(3-fluor-5,6,7,8-tetrahydro-5,5,8,8-tetramethyl-2-naphthyl)-1-propenyl]benzylester
Phosphorsäure-4-[2-(5,6,7,8-tetrahydro-1-methoxy-3,5,5,8,8-pentamethyl-2-naphthyl)-1-propenyl]benzylester
Phosphorsäure-4-[2-(5,6,7,8-tetrahydro-1-methoxy-4,5,5,8,8-pentamethyl-2-naphthyl)-1-propenyl]benzylester
Phosphorsäure-4-[2-(5,6,7,8-tetrahydro-1-hydroxy-3,5,5,8,8-pentamethyl-2-naphthyl)-1-propenyl]benzylester
Phosphorsäure-4-[2-(1-hexoxy-5,6,7,8-tetrahydro-5,5,8,8-tetramethyl-2-naphthyl)-1-propenyl]benzylester
Phosphorsäure-4-[2-(1-ethoxy-5,6,7,8-tetrahydro-3,5,5,8,8-pentamethyl-2-naphthyl)-1-propenyl]benzylester
Phosphorsäure-4-[2-(5,6,7,8-tetrahydro-1,3,5,5,8,8-hexamethyl-2-naphthyl)-1-propenyl]benzylester
Phosphorsäure-4-[2-(5,6,7,8-tetrahydro-1,4,5,5,8,8-hexamethyl-2-naphthyl)-1-propenyl]benzylester
Phosphorsäure-4-[2-(5,6,7,8-tetrahydro-1,4-dimethoxy-3,5,5,8,8-hexamethyl-2-naphthyl)-1-propenyl]benzylester
Phosphorsäure-4-[2-(5,6,7,8-tetrahydro-8,8-dimethyl-2-naphthyl)-1-propenyl]benzylester

Phosphorsäure-4-[2-(5,6,7,8-tetrahydro-3,8,8-trimethyl-2-naphthyl)-1-propenyl]benzylester
Phosphorsäure-4-[2-(5,6,7,8-tetrahydro-3,8,8-trimethyl-2-naphthyl)-1-ethenyl]benzylester
Phosphorsäure-4-[2-(5,6,7,8-tetrahydro-5,5,8,8-tetramethyl-2-naphthyl)-1-ethenyl]benzylester
Phosphorsäure-4-[2-(5,6,7,8-tetrahydro-3-methoxy-5,5,8,8-tetramethyl-2-naphthyl)-1-ethenyl]benzylester
Phosphorsäure-4-[2-(5,6,7,8-tetrahydro-1,3-dimethoxy-5,5,8,8-tetramethyl-2-naphthyl)-1-ethenyl]benzylester
Phosphorsäure-4-[2-(5,6,7,8-tetrahydro-1,4-dimethoxy-5,5,8,8-tetramethyl-2-naphthyl)-1-ethenyl]benzylester
Phosphorsäure-4-[2-(5,6,7,8-tetrahydro-1-methoxy-3,5,5,8,8-pentamethyl-2-naphthyl)-1-ethenyl]benzylester
Phosphorsäure-4-[2-(5,6,7,8-tetrahydro-1-hydroxy-3,5,5,8,8-pentamethyl-2-naphthyl)-1-ethenyl]benzylester
Phosphorsäure-4-[2-(5,6,7,8-tetrahydro-5,5,8,8-tetramethyl-2-naphthyl)-1-buten(1)-yl]benzylester
Phosphorsäure-4-[2-(5,6,7,8-tetrahydro-5,5,8,8-tetramethyl-2-naphthyl)-1-octen(1)-yl]benzylester
Phosphorsäure-4-[2-cyclopropyl-(5,6,7,8-tetrahydro-5,5,8,8-tetramethyl-2-naphthyl)-1-ethenyl]benzylester
Phosphorsäure-4-[3-methyl-2-(5,6,7,8-tetrahydro-5,5,8,8-tetramethyl-2-naphthyl)-1-buten(1)-yl]benzylester
Phosphorsäure-4-[2-(5,6,7,8-tetrahydro-3,5,5,8,8-pentamethyl-2-naphthyl)-1-buten(1)-yl]benzylester
Phosphorsäure-4-[2-(2,3-dihydro-1,1,2,3,3,6-hexamethyl-5(1H)-indenyl)-1-ethyl] benzylester
Phosphorsäure-4-[2-(2,3-dihydro-1,1,3,3,6-pentamethyl-5(1H)-indenyl)-1-propenyl]benzylester
Phosphorsäure-4-[2-(2,3-dihydro-1,1,3,3,6-pentamethyl-5(1H)-indenyl)-1-ethenyl]benzylester
Phosporsäure-4-[2-(2,3-dihydro-1,1,2,3,3,6-hexamethyl-5-(1H)-indenyl)-1-propenyl]benzylester
Phosphorsäure-4-[2-(2,3-dihydro-1,1,2,3,3-pentamethyl-6-propyl-5(1H)-indenyl)-1-propenyl]benzylester
Phosphorsäure-4-[2-(2,3-dihydro-1,1,2,3,3-pentamethyl-5(1H)-indenyl)-1-ethenyl]benzylester
Phosphorsäure-1-[4-[2-(5,6,7,8-tetrahydro-5,5,8,8-tetramethyl-2-naphthyl)-1-propenyl]phenyl]propylester
Phosphorsäure-1-[4-[2-(5,6,7,8-tetrahydro-5,5,8,8-tetramethyl-2-naphthyl)-1-propenyl]phenyl]pentylester
Phosphorsäure-1-[4-[2-(5,6,7,8-tetrahydro-5,5,8,8-tetramethyl-2-naphthyl)-1-propenyl]phenyl]ethylester
Phosphorsäure-[2-methyl-1-[4-[2-(5,6,7,8-tetrahydro-5,5,8,8-tetramethyl-2-naphthyl)-1-propenyl]phenyl]ethyl]ester
Phosphorsäure-4-[2-(5,6,7,8-tetrahydro-5,5,8,8-tetramethyl-2-naphthyl)-1-propenyl]benzyldimethylester
Phosphorsäure-4-[2-(5,6,7,8-tetrahydro-5,5,8,8-tetramethyl-2-naphthyl)-1-propenyl]benzyldiethylester
Phosphorsäure-4-[2-(5,6,7,8-tetrahydro-5,5,8,8-tetramethyl-2-naphthyl)-1-propenyl]benzyldibutylester
Phosphorsäure-4-[2-(5,6,7,8-tetrahydro-5,5,8,8-tetramethyl-2-naphthyl)-1-propenyl]benzyldioctylester
Phosphorsäure-4-[2-(5,6,7,8-tetrahydro-5,5,8,8-tetramethyl-2-naphthyl)-1-propenyl]benzyldibenzylester
Phosphorsäure-4-[2-(5,6,7,8-tetrahydro-5,5,8,8-tetramethyl-2-naphthyl)-1-propenyl]benzyldiphenylester
Phosphorsäure-4-[2-(5,6,7,8-tetrahydro-5,5,8,8-tetramethyl-2-naphthyl)-1-propenyl]benzylmethylester
Phosphorsäure-4-[2-(5,6,7,8-tetrahydro-5,5,8,8-tetramethyl-2-naphthyl)-1-ethenyl]benzyldiethylester
Phosphorsäure-4-[2-(2,3-dihydro-1,1,2,3,3-pentamethyl-5(1H)-indenyl)-1-propenyl]benzyldiethylester
Phosphorsäure-4-[2-(5,6,7,8-tetrahydro-5,5,8,8-tetramethyl-2-naphthyl)-1-propenyl]benzylester-diamid
Phosphorsäure-4-[2-(5,6,7,8-tetrahydro-5,5,8,8-tetramethyl-2-naphthyl)-1-propenyl]benzylester-bis(methylamid)
Phosphorsäure-4-[2-(5,6,7,8-tetrahydro-5,5,8,8-tetramethyl-2-naphthyl)-1-propenyl]benzylester-bis(dimethylamid)
Phosphorsäure-4-[2-(5,6,7,8-tetrahydro-5,5,8,8-tetramethyl-2-naphthyl)-1-propenyl]benzylester-bis(diethylamid)
Phosphorsäure-4-[2-(5,6,7,8-tetrahydro-5,5,8,8-tetramethyl-2-naphthyl)-1-propenyl]benzylester-bis-morpholid

Die Verbindung der Formel I, in denen $R^8$ und/oder $R^9$ OH-Gruppen bedeuten, können mit 1 bzw. 2 Mol Base Salze bilden. Geeignete Salze sind beispielsweise Ammonium-, Alkalimetall-, insbesondere des Natriums, Kaliums und Lithiums, Erdalkalimetallsalze, insbesondere des Calciums oder Magnesiums sowie Salze mit geeigneten organischen Basen, wie mit niederen Alkylaminen.z. B. Methylamin oder Ethylamin, insbesondere Cyclohexylamin, mit substituierten niederen Alkylaminen, insbesondere hydroxysubstituierten Alkylaminen, wie Diethanolamin, Triethanolamin oder Tris-hydroxymethyl)-aminomethan, Piperidin oder Morpholin.

Die neuen Phosphorsäurevinylbenzylester lassen sich herstellen, indem man

a) einen Alkohol der Formel II

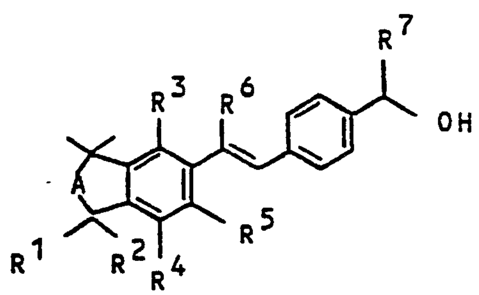

II,

worin A und $R^1$-$R^7$ die oben angegebene Bedeutung haben, mit einem Phosphorylierungsreagenz der Formel III

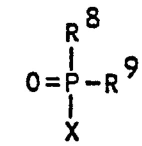 III,

worin $R^8$ und $R^9$ die angegebene Bedeutung haben, und X für ein Halogenatom, den Imidazol-, N,N'-Dicyclohexylisoharnstoff-, Trichloracetimidat-, p-Toluolsulfonyloxy-, P-Nitrophenyloxy- oder Mesityloxyrest oder für die Gruppe -O-P(O)$R^8R^9$ steht, oder

b) einen Alkohol der Formel II mit einem Phosphorylierungsreagenz der Formel V

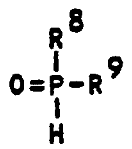 V,

worin $R^8$ und $R^9$ die angegebene Bedeutung haben unter oxidativen Bedingungen umsetzt

und die so erhaltenen Verbindungen gegebenenfalls in ihre physiologisch verträglichen Selze überführt.

Die Umsetzung a) findet üblicherweise bei Temperaturen von -20 bis 100°C statt, je nach Art des Phosphorylierungsreagenzes.

Die Säuren, die bei der Reaktion entstehen, z. B. Halogenwasserstoffe, werden zweckmäßigerweise mit Basen gebunden, wobei vor allem tertiäre Stickstoffbasen wie Pyridin, N,N-Dimethylanilin, 2-Picolin, 2,6-Lutidin, Imidazol oder Triethylamin, oder Hydride oder Carbonate der Alkalimetalle, vorzugsweise des Natriums und des Kaliums, Verwendung finden. Als säurebindendes Mittel kann außerdem Arcylnitril fungieren.

Die Umsetzungen werden üblicherweise in Gegenwart eines Verdünnungsmittels durchgeführt, beispielsweise Kohlenwasserstoffe wie Benzol, Toluol, Xylol oder Petrolether, polar aprotische Lösungsmittel wie Aceton, 2-Butanon, Acetonitril, Dimethylformamid oder Dimethylsulfoxid, oder Ether wie Tetrahydrofuran, Dioxan oder Dimethoxyethan. Auch die oben genannten tertiären Stickstoffbasen können, im Überschuß, eingesetzt, als Lösungsmittel dienen.

Wenn von den Resten $R^8$ oder $R^9$ einer oder beide für eine Hydroxylgruppe stehen, wird vorzugsweise die Trichloracetimidatmethode verwendet, wobei also X in Formel (III) für den Trichloracetimidatrest steht. Man setzt dabei einen Alkohol der Formel (I) mit wasserfreier Phosphorsäure bzw. mit einem Phosphorsäureester der Formel (III), worin X für eine Hydroxylgruppe steht, mit Trichloracetonitril in Gegenwart einer Base wie z. B. Triethylamin bei einer Temperatur bis zu 75°C, gegebenenfalls in einem Lösungsmittel wie Acetonitril, um, wobei die Trichloracetimidate der Formel (III) in situ gebildet werden.

Des Verfahren b) wird vorzugsweise in Tetrachlorkohlenstoff und 50 %-iger Natronlauge unter transferkatalytischen Bedingungen durchgeführt. Als Katalysatoren dienen insbesondere quartäre Ammoniumsalze wie Tetrabutylammoniumbromid.

Die nach den angegebenen Verfahren hergestellten erfindungsgemäßen Verbindungen der Formel (I), worin die Reste $R^8$ und/oder $R^9$ für Hydroxylgruppen stehen, werden vorteilhaft zunächst als Salze, vorzugsweise als Mono- oder Bis-cyclohexylammoniumphosphate isoliert, aus denen die freien Phosphorsäuremono- oder -diester durch Behandeln mit verdünnten Mineralsäuren oder mittels eines sauren Ionenaustauschers freigesetzt werden können.

Die als Ausgangsverbindungen benutzten Alkohole der Formel (II) werden durch Reduktion der Carbonsäuren oder Carbonsäureester der Formel (IV)

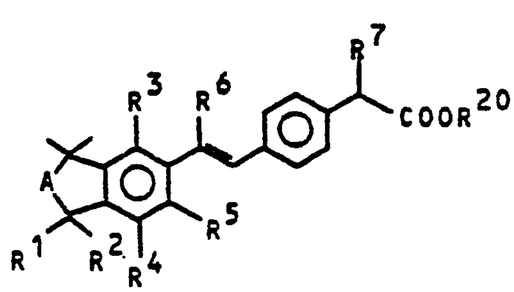 IV,

worin A, $R^1$, $R^2$, $R^3$, $R^4$, $R^5$, $R^6$ und $R^7$ die oben angegebene Bedeutung haben, und $R^{20}$ für Wasserstoff oder einen $C_{1-4}$-Alkylrest steht, erhalten. Verbindungen der Formel (IV) sind in der DE-CS 28 94 354 beschrieben oder können nach dem dort beschriebenen Verfahren hergestellt werden.

Die erfindungsgemäßen Verbindungen und ihre physiologisch verträglichen Salze können aufgrund ihrer

pharmakologischen Eigenschaften bei der topischen und systemischen Therapie und auch Prophylaxe von Preakanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Bevorzugte Inidikationsgebiete sind neben der Therapie von dermatologischen Erkrankungen die prophylaktische und therapeutische Behandlung von Praekanzerosen und Tumoren sowie die Behandlung arthritischer Erkrankungen. Die dermatologische Aktivität, beispielsweise für die Behandlung von Akne, kann u.a. durch die Bestimmung der komedolytischen Aktivität und die Fähigkeit nachgewiesen werden, die Anzahl der Utrikuli im Modell der Rhino-Maus zu reduzieren. Diese Methode ist von L.H. Kligman et al. in The Journal of Investigative Dermatology 73, 354-358 (1979) und von J.A. Mezick et al in "Methods of Dermatology" (Ed. Maibach, Lowe) Vol. 2, S. 59-63, Karger, Basel 1985 beschrieben. Die antiarthritische Wirkung der erfindungsgemäßen Verbindungen kann in üblicher Weise im Tierexperiment im Adjuvans-Arthritis-Modell bestimmt werden. Die präventive Wirkung bei der Entstehung und Entwicklung prämaligner Läsionen können beispielsweise in den nachfolgenden Testmodellen aufgezeigt werden. Die erfindungsgemäßen Verbindungen haben an Hamstertreachealgewebe in vitro die nach Vitamin-A-Mangel einsetzende Keratinisierung auf. Diese Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964 bis 972 (1976) oder aus Nature 250, 64-66 (1974) und Nature 253, 47 bis 50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15 bis 22 (1978) und Proc. Natl. Acad. Sci. USA 77, 2936-2940. (1980) entnommen werden. Weiterhin sei auf die Bestimmung der antagonistischen Wirkungen gegenüber Phorbolester verwiesen, wie in Cancer Res. 37, 2196-2201 (1977) beschrieben wird.

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutischtechnischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derertige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %-iger Konzentration, bevorzugt in 0,001 bis 0,1 %-iger Konzentration und bei systemischer Anwendung vorzugsweise in einer Einzeldosis von 0,1 bis 50 mg enthalten und täglich in einer oder mehreren Dosierungen je nach Art und Schwere der Erkrankung verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostesrat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleitmittel usw. zugesetzt werden. Voraussetzung ist, daß alle bei der Herstellung pharmazeutische Zubereitung verwendeten Stoffe toxikologisch unbedenklich sind und mit den verwendeten Wirkstoffen verträglich sind.

Herstellung der erfindungsgemäßen Verbindungen

**Beispiel 1**

Phosphorsäure-4-[2-(5,6,7,8-tetrahydro-5,5,8,8-tetramethyl-2-naphthyl)-1-propenyl]benzylester, Monocyclohexylammoniumsalz.

Zu 25,3 g (75,7 mmol) 4-[2-(5,6,7,8-Tetrahydro-5,5,8,8-tetramethyl-2-naphthyl)-1-propenyl]benzylalkohol und 16,5 g (0,15 mol) Trichloracetonitril wurde eine Lösung aus 6,06 g (0,06 mol) wasserfreier Phosphorsäure, 12,2 g (0,12 mol) Triethylamin, 45,3 g Acetonitril und 0,45 ml Wasser innerhalb 55 min zugetropft. Man erwärmte kurz

auf 50° C und ließ über Nacht bei Raumtemperatur rühren.

Man goß auf 75 ml Wasser, extrahierte mit Ether, bis sich die Etherphase nicht mehr gelb färbte (ca. 5 x 75 ml Ether), und engte die Wasserphase bei einer Badtemperatur von 30 bis 40°C ein. Man versetzte mit 6 g Cyclohexylamin und 60 ml Aceton, ließ das Gemisch über Nacht im Tiefkühlschrank stehen und filtrierte das entstandene Kristallisat ab. Nach Trocknen im Vakuumtrockenschrank bei 30° C über Phosphorpentoxid erhielt man 16 g Rohprodukt.

5 g dieses Rohprodukts wurden in 100 ml eines 1 : 1 Gemisches aus Chloroform und Wasser weitgehend gelöst. Von unlöslichen Betandteilen wurde abfiltriert, und das milchig trübe Filtrat 2 Tage stehen gelassen, wobei ein Großteil des Chloroforms verdampfte und sich ein farbloser kristalliner Niederschlag bildete, der abfiltriert wurde. Nach Trocknen über Phosphorpentoxid wurden 1,5 g der Titelverbindung, Schmp. 235 - 239° C, erhalten.

**Beispiel 2**

Phosphorsäure-4-[2-(2,3-dihydro-1,1,2,3,3-pentamethyl-5(1H)-indenyl)-1-propenyl]benzylester, Biscyclohexylammoniumsalz

Analog Beispiel 1 erhielt man aus 10 g (29 mmol) 4-[2-(2,3-dihydro-1,1,2,3,3-pentamethyl-5(1H)-indenyl)-1-propenyl]-benzylalkohol, 2,2 g (23 mmol) Phosphorsäure, 8,2 g (57 mmol) Trichloracetonitril und 4,5 g (45 mmol) Cyclohexylamin, 2,2 g der Titelverbindung, Schmp. 206 - 210° C.

**Beispiel 3**

Phosphorsäure-4-[2-(5,6,7,8-tetrahydro-5,5,8,8-tetramethyl-2-naphthyl)-1-propenyl]benzyldiethylester

Zu einem Gemisch aus 7 g (20 mmol) 4-[2-(5,6,7,8-Tetrahydro-5,5,8,8-tetramethyl-2-naphthyl)-1-propenyl]benzylalkohol, 12 ml Tetrachlorkohlenstoff, 0,16 g Tetrabutylammoniumbromid und 6 ml 50 %-ige Natronlauge tropfte man unter Eiskühlung eine Lösung von 3,5 g (25 mmol) Diethylphosphit in 6 ml Tetrachlorkohlenstoff zu. Man ließ 3 Tage bei Raumtemperatur nachrühren. Man extrahierte mit 30 ml Methylenchlorid, wusch die organische Phase mit 10 ml 2 %-iger Salzsäure und zweimal 10 ml Wasser, trocknete ($Na_2SO_4$) und engte ein. Der Rückstand wurde aus Pentan und nochmals aus Petrolether umkristallisiert. Man erhielt so 0,3 g der Titelverbindung, Schmp. 70 - 74°C, 98 % Reinheit nach HPLC (C 18 reversed phase; Hexan/Ethanol/0,1 % Essigsäure; 100 ml/min.).

**Patentansprüche** für dei Vertragsstaaten BE, CH, DE, FR, GB, IT, LI, NL, SE

1. Phosphorsäurevinylbenzylester der Formel I

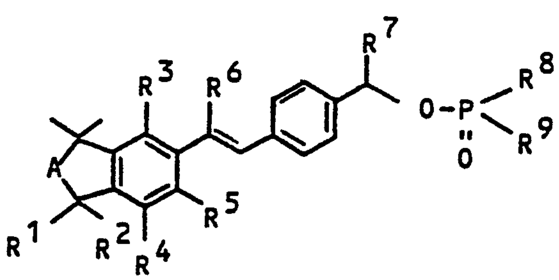

I,

worin

A einen gegebenenfalls durch $C_{1-4}$-Alkylgruppen substituierten Methylen- oder Ethylenrest,
$R^1$ und $R^2$ Wasserstoffatome oder Methylgruppen
$R^3$ ein Wasserstoffatom, eine Methyl-, Hydroxy- oder $C_{1-6}$-Alkoxygruppe
$R^4$ und $R^5$ Wasserstoffatome, $C_{1-4}$-Alkyl- oder Methoxygruppen oder Halogenatome
$R^6$ ein Wasserstoffatom, eine $C_{1-6}$-Alkyl- oder $C_{3-6}$-Cycloalkylgruppe
$R^7$ ein Wasserstoffatom oder eine $C_{1-4}$-Alkylgruppe und
$R^8$ und $R^9$ die Reste $-OR^{10}$ oder $-NR^{11}R^{12}$ bedeuten, worin
$R^{10}$ ein Wasserstoffatom oder eine $C_{1-8}$-Alkyl-, Phenyl- oder Benzylgruppe,
$R^{11}$ und $R^{12}$ Wasserstoffatome, $C_{1-6}$-Alkylgruppen oder zusammen mit dem Stickstoffatom, an das sie

gebunden sind, einen Pyrrolidino-, Piperidino- oder Morpholino-Rest bedeuten und wobei jeweils zwei Reste $R^{10}$, $R^{11}$ und/oder $R^{12}$ zusammen auch für eine Ethylen- oder Propylengruppe stehen können, sowie deren physiologisch verträglichen Salze.

2. Phosphorsäurevinylbenzylester der Formel I gemäß Anspruch 1, dadurch gekennzeichnet, daß
A einen gegebenenfalls durch eine Methylgruppe substituierten Methylen- oder Ethylenrest darstellt,
$R^1$ und $R^2$ die angegebene Bedeutung besitzen,
$R^3$, $R^4$ und $R^5$ Wasserstoffatome,
$R^6$ ein Wasserstoffatom oder eine Methylgruppe,
$R^7$ Wasserstoff und
$R^8$ und $R^9$ eine Hydroxy- oder $C_{1-4}$-Alkoxygruppe bedeuten.

3. Verfahren zur Herstellung der Phosphorsäurevinylbenzylester der Formel I gemäß Anspruch 1, <u>dadurch gekennzeichnet</u>, das man
a) einen Alkohol der Formel II

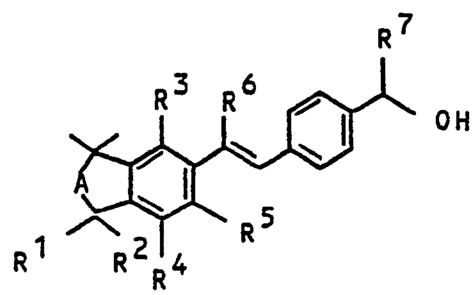

II,

worin A und $R^1$-$R^7$ die im Anspruch 1 angegebene Bedeutung haben, mit einem Phosphorylierungsreagenz der Formel III

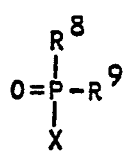

III,

worin $R^8$ und $R^9$ die im Anspruch 1 angegebene Bedeutung haben, und X für ein Halogenatom, den Imidazol-, N,N'-Dicyclohexylisoharnstoff-, Trichloracetimidat-, p-Toluolsulfonyloxy-, p-Nitrophenyloxy- oder Mesityloxyrest oder für die Gruppe -O-P(O)$R^8R^9$ steht, oder
b) einen Alkohol der Formel II mit einem Phosphorylierungsreagenz der Formel V

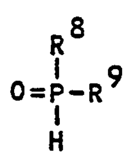

V,

worin $R^8$ und $R^9$ die im Anspruch 1 angegebene Bedeutung haben, unter oxidativen Bedingungen umsetzt und die so erhaltenen Verbindungen gegebenenfalls in ihre physiologisch verträglichen Salze überführt.

4. Phosphorsäurevinylbenzylester der Formel I gemäß Anspruch 1 zur Verwendung bei der Bekämpfung von Krankheiten.

**Patentansprüche** für den Vertragsstaat AT

1. Verfahren zur Herstellung der Phosphorsäurevinylbenzylester der Formel I

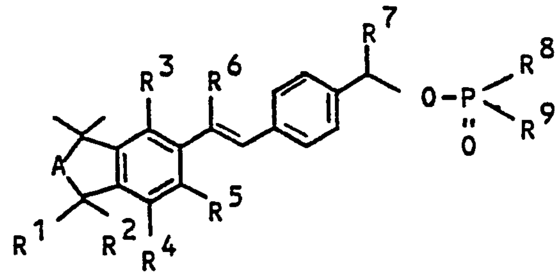

I,

worin

A einen gegebenenfalls durch $C_{1-4}$-Alkylgruppen substituierten Methylen- oder Ethylenrest,
$R^1$ und $R^2$ Wasserstoffatome oder Methylgruppen
$R^3$ ein Wasserstoffatom, eine Methyl-, Hydroxy- oder $C_{1-6}$-Alkoxygruppe
$R^4$ und $R^5$ Wasserstoffatome, $C_{1-4}$-Alkyl- oder Methoxygruppen oder Halogenatome
$R^6$ ein Wasserstoffatom, eine $C_{1-6}$-Alkyl- oder $C_{3-6}$-Cycloalkylgruppe
$R^7$ ein Wasserstoffatom oder eine $C_{1-4}$-Alkylgruppe und
$R^8$ und $R^9$ die Reste $-OR^{10}$ oder $-NR^{11}R^{12}$ bedeuten, worin
$R^{10}$ ein Wasserstoffatom oder eine $C_{1-8}$-Alkyl-, Phenyl- oder Benzylgruppe,
$R^{11}$ und $R^{12}$ Wasserstoffatome, $C_{1-6}$-Alkylgruppen oder zusammen mit dem Stickstoffatom, an das sie gebunden sind, einen Pyrrolidino, Piperidino- oder Morpholino-Rest bedeuten und wobei jeweils zwei Reste
$R^{10}$, $R^{11}$ und/oder $R^{12}$ zusammen auch für eine Ethylen oder Propylengruppe stehen können,
sowie deren physiologisch verträgliche Salze, dadurch gekennzeichnet, daß man
a) einen Alkohol der Formel II

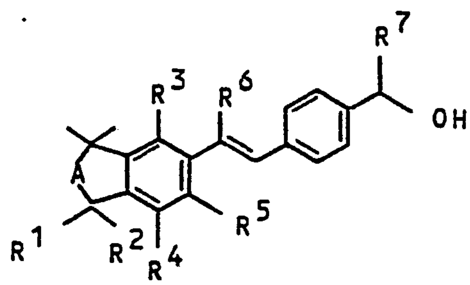

II,

worin A und $R^1$-$R^7$ die oben angegebene Bedeutung haben, mit einem Phosphorylierungsreagenz der Formel III

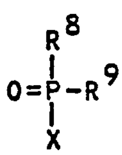

III,

worin $R^8$ und $R^9$ die angegebene Bedeutung haben, und X für ein Halogenatom, den Imidazol-, N,N'-Dicyclohexylisoharnstoff-, Trichloracetimidat-, p-Toluolsulfonyloxy-, p-Nitrophenyloxy- oder Mesityloxyrest oder für die Gruppe $-O-P(O)R^8R^9$ steht, oder
b) einen Alkohol der Formel II mit einem Phosphorylierungsreagenz der Formel V

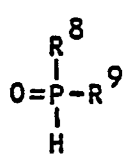

V,

worin $R^8$ und $R^9$ die angegebene Bedeutung haben unter oxidativen Bedingungen umsetzt
und die so erhaltenen Verbindungen gegebenenfalls in ihre physiologisch verträglichen Salze überführt.

2. Verfahren gemäß Anspruch 1, dadurch gekennzeichnet, daß man Verbindungen der Formel I gemäß Anspruch 1 herstellt, in der

A einen gegebenenfalls durch eine Methylgruppe substituierten Methylen- oder Ethylenrest darstellt,
$R^1$ und $R^2$ die angegebene Bedeutung besitzen,
$R^3$, $R^4$ und $R^5$ Wasserstoffatome,
$R^6$ ein Wasserstoffatom oder eine Methylgruppe
$R^7$ Wasserstoff und
$R^8$ und $R^9$ eine Hydroxy- oder $C_{1-4}$-Alkoxygruppe bedeuten.

**Revendications** pour les Etat contractants BE, CH, DE, FR, GB, IT, LI, NL, SE

1. Esters d'acides vinyl-benzyl-phosphoriques de la formule I

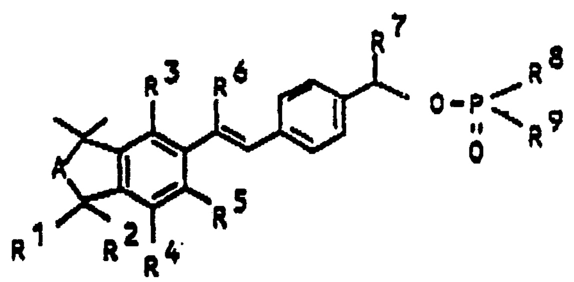

I,

dans laquelle

A désigne un groupe méthylène ou éthylène éventuellement substitué par des radicaux alkyle en $C_1$ à $C_4$,
$R^1$ et $R^2$ désignent des atomes d'hydrogène ou des radicaux méthyle,
$R^3$ désigne un atome d'hydrogène ou un radical méthyle, hydroxy ou alcoxy en $C_1$ à $C_6$,
$R^4$ et $R^5$ dé signent des atomes d'hydrogène ou d'halogène ou des groupes alkyle en $C_1$ à $C_4$ ou méthoxy,
$R^6$ désigne un atome d'hydrogène, un radical alkyle en $C_1$ à $C_6$ ou un groupe cycloalkyle en $C_3$ à $C_6$,
$R^7$ désigne un atome d'hydrogène ou un radical alkyle en $C_1$ à $C_4$ et
$R^8$ et $R^9$ représentent des groupes $-OR^{10}$ ou $-NR^{11}R^{12}$, où
$R^{10}$ est un atome d'hydrogène ou un groupe alkyle en $C_1$ à $C_8$, phényle ou benzyle et
$R^{11}$ et $R^{12}$ désignent des atomes d'hydrogène ou des radicaux alkyle en $C_1$ à $C_6$ ou forment avec l'atome d'azote adjacent un groupe pyrrolidino, pipéridino ou morpholino, ou
deux des restes $R^{10}$, $R^{11}$ et(ou) $R^{12}$ forment ensemble un groupe éthylène ou propylène,
ainsi que leurs sels physiologiquement compatibles.

2. Esters d'acides vinyl-benzyl-phosphoriques de la formule I suivant la revendication 1, caractérisés en ce que:

A désigne un groupe méthylène ou éthylène éventuellement substitué par un radical méthyle,
$R^1$ et $R^2$ possèdent les significations définies,
$R^3$, $R^4$ et $R^5$ sont des atomes d'hydrogène,
$R^6$ désigne un atome d'hydrogène ou un radical méthyle,
$R^7$ = H et
$R^8$ et $R^9$ désignent un radical hydroxy ou alcoxy en $C_1$ à $C_4$.

3. Procédé de préparation d'esters d'acides vinylbenzyl-phosphoriques de la formule I suivant la revendication 1, caractérisé en ce que l'on fait réagir:

soit a) un alcool de la formule II

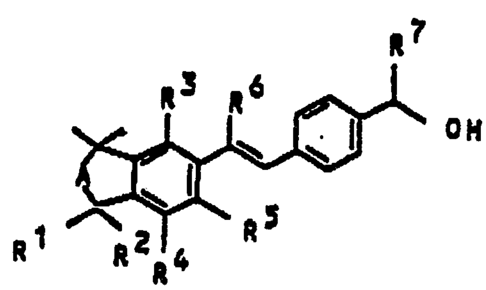

II,

dans laquelle A et $R^1$ à $R^7$ possèdent la signification définie dans la revendication 1, avec un agent de phosphorylation de la formule III

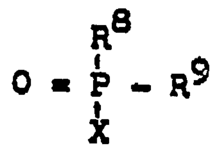

III,

dans laquelle $R^8$ et $R^9$ possèdent la signification définie dans la revendication 1 et X représente un atome d'halogène ou un groupe imidazole, N, N'-dicyclohexyl-isourée, trichloracétimidate, p-toluène-sulfonyloxy, p-nitrophényloxy ou mésityloxy ou un groupe -O-P(O)$R^8R^9$,

soit b) un alcool de la formule II, dans des conditions oxydantes, avec un agent de phosphorylation de la formule V

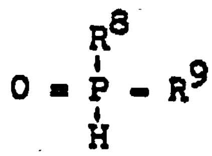

V,

dans laquelle $R^8$ et $R^9$ possèdent la signification définie dans la revendication 1,

puis on transforme éventuellement les composés obtenus en leurs sels physiologiquement compatibles.

4. Esters d'acides vinyl-benzyl-phosphoriques de la formule I suivant la revendication 1 pour le traitement de maladies.

**Revendications** pour l'Etat contractant AT

1. Procédé de préparation d'esters d'acides vinylbenzyl-phosphoriques de la formule I

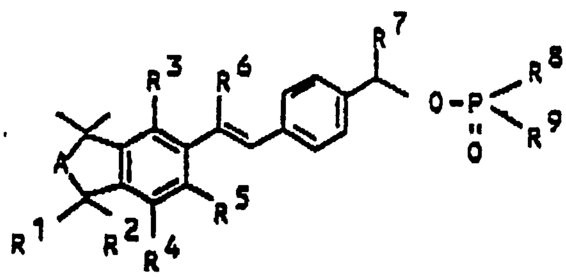

I,

dans laquelle

A désigne un groupe méthylène ou éthylène éventuellement substitué par des radicaux alkyle en $C_1$ à $C_4$,
$R^1$ et $R^2$ désignent des atomes d'hydrogène ou des radicaux méthyle,
$R^3$ désigne un atome d'hydrogène ou un radical méthyle, hydroxy ou alcoxy en $C_1$ à $C_6$,
$R^4$ et $R^5$ désignent des atomes d'hydrogène ou d'halogène ou des groupes alkyle en $C_1$ à $C_4$ ou méthoxy,
$R^6$ désigne un atome d'hydrogène, un radical alkyle en $C_1$ à $C_6$ ou un groupe cycloalkyle en $C_3$ à $C_6$,
$R^7$ désigne un atome d'hydrogène ou un radical alkyle en $C_1$ à $C_4$ et
$R^8$ et $R^9$ représentent des groupes -$OR^{10}$ ou -$NR^{11}$ $R^{12}$ où
$R^{10}$ est un atome d'hydrogène ou un groupe alkyle en $C_1$ à $C_8$, phényle ou benzyle et
$R^{11}$ et $R^{12}$ désignent des atomes d'hydrogène ou des radicaux alkyle en $C_1$ a $C_6$ ou forment avec l'atome d'azote adjacent un groupe pyrrolidino, pipéridino ou morpholino, ou
deux des restes $R^{10}$, $R^{11}$ et(ou) $R^{12}$ forment ensemble un groupe éthylène ou propylène,
ainsi que de leurs sels physiologiquement compatibles, caractérisé en ce que l'on fait réagir:

soit a) un alcool de la forme II

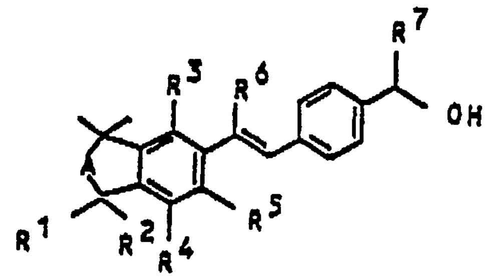

II,

dans laquelle A et $R^1$ à $R^7$ possedent la signification définie dans la revendication 1, avec un agent de phosphorylation de la formule III

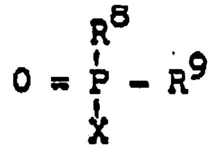

III,

dans laquelle $R^8$ et $R^9$ possèdent la signification définie dans la revendication 1 et X représente un atome d'halogène ou un groupe imidazole, N, N'-dicyclohexyl-isourée, trichloracétimidate, p-toluène-sulfonyloxy, p-nitrophényloxy ou mésityloxy ou un groupe -O-P(O)$R^8R^9$,

soit b) un alcool de la formule II, dans des conditions oxydantes, avec un agent de phosphorylation de la formule V

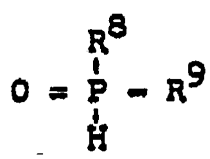

V,

dans laquelle $R^8$ et $R^9$ possèdent la signification definie dans la revendication 1,

puis on transforme éventuellement les composés obtenus en leurs sels physiologiquement compatibles.

2. Procédé suivant la revendication 1, caractérisé en ce qu'il sert à préparer des composés de la formule I suivant la revendication 1, pour lesquels

A désigne un groupe méthylène ou éthylène éventuellement substitué par un radical méthyle,

$R^1$ et $R^2$ possèdent la signification définie,

$R^3$, $R^4$ et $R^5$ sont des atomes d'hydrogène,

$R^6$ désigne un atome d'hydrogène ou un radical méthyle,

$R^7$ = H et

$R^8$ et $R^9$ désignent un radical hydroxy ou alcoxy en $C_1$ à $C_4$.

**Claims** for the Contracting States BE, CH, FR, GB, IT, LI, NL, SE

1. A vinylbenzyl phosphate of the formula I

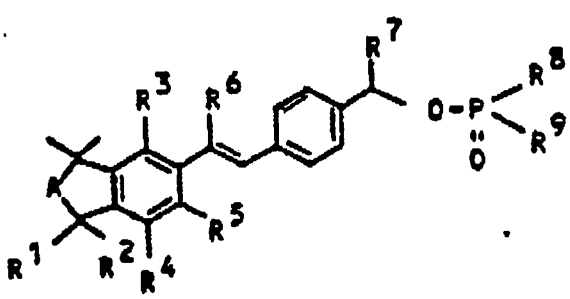

I

where

A is a methylene or ethylene radical which is unsubstituted or substituted by $C_1$-$C_4$-alkyl, $R^1$ and $R^2$ are each

hydrogen or methyl, $R^3$ is hydrogen, methyl, hydroxyl or $C_1$-$C_6$-alkoxy, $R^4$ and $R^5$ are each hydrogen, $C_1$-$C_4$-alkyl, methoxy or halogen, $R^6$ is hydrogen, $C_1$-$C_5$-alkyl or $C_3$-$C_5$-cycloalkyl, $R^7$ is hydrogen or $C_1$-$C_4$-alkyl and $R^8$ and $R^9$ are each a radical $-OR^{10}$ or $-NR^{11}R^{12}$, where $R^{10}$ is hydrogen, $C_1$-$C_8$-alkyl, phenyl or benzyl, $R^{11}$ and $R^{12}$ are each hydrogen or $C_1$-$C_6$-alkyl, or $R^{11}$ and $R^{12}$, together with the nitrogen atom to which they are bonded, form a pyrrolidino, piperidino or morpholino radical, and two radicals $R^{10}$, $R^{11}$ and/or $R^{12}$ together in each case may furthermore be ethylene or propylene, and its physiologically tolerated salts.

2. A vinylbenzyl phosphate of the formula I as set forth in claim 1, where A is a methylene or ethylene radical which is unsubstituted or substituted by methyl, $R^1$ and $R^2$ have the above meanings, $R^3$, $R^4$ and $R^5$ are each hydrogen, $R^6$ is hydrogen or methyl, $R^7$ is hydrogen and $R^8$ and $R^9$ are each hydroxyl or $C_1$-$C_4$-alkoxy.

3. A process for the preparation of a vinylbenzyl phosphate of the formula I as claimed in claim 1, wherein

a) an alcohol of the formula II

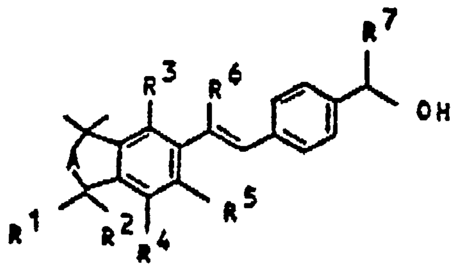

II

where A, $R^1$, $R^2$, $R^3$, $R^4$, $R^5$, $R^6$ and $R^7$ are each as defined in claim 1, is reacted with a phosphorylating reagent of the formula III

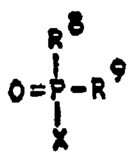

III

where $R^8$ and $R^9$ are each as defined in claim 1 and X is halogen or an imidazole, N,N-dicyclohexylisourea, trichloroacetimidate, p-toluenesulfonyloxy, p-nitrophenyloxy or mesityloxy radical or a group $-O-P(O)R^8R^9$, or

b) an alcohol of the formula II is reacted under oxidative conditions with a phosphorylating reagent of the formula V

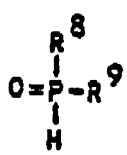

V,

where $R^8$ and $R^9$ are each as defined in claim 1,

and, if desired, the resulting compound is converted to one of its physiologically tolerated salts.

4. A vinylbenzyl phosphate of the formula I as claimed in claim 1 for use in the treatment of disorders.

**Claims** for the Contracting State AT

1. A process for the preparation of a vinylbenzyl phosphate of the formula I

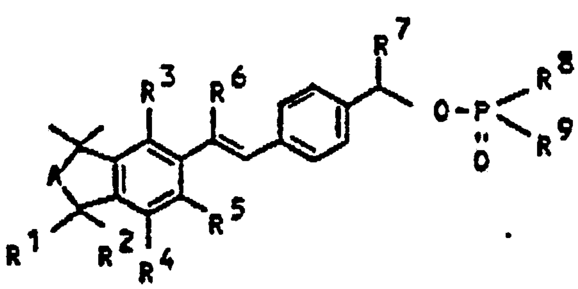

I

where A is a methylene or ethylene radical which is unsubstituted or substituted by $C_1$-$C_4$-alkyl, $R^1$ and $R^2$ are each hydrogen or methyl, $R^3$ is hydrogen, methyl, hydroxyl or $C_1$-$C_6$-alkoxy, $R^4$ and $R^5$ are each hydrogen, $C_1$-$C_4$-alkyl, methoxy or halogen, $R^6$ is hydrogen, $C_1$-$C_6$-alkyl or $C_3$-$C_5$-. cycloalkyl, $R^7$ is hydrogen or $C_1$-$C_4$-alkyl and $R^8$ and $R^9$ are each a radical $-OR^{10}$ or $-NR^{11}R^{12}$, where $R^{10}$ is hydrogen, $C_1$-$C_8$-alkyl, phenyl or benzyl, $R^{11}$ and $R^{12}$ are each hydrogen or $C_1$-$C_6$-alkyl, or $R^{11}$ and $R^{12}$, together with the nitrogen atom to which they are bonded, form a pyrrolidino, piperidino or morpholino radical, and two radicals $R^{10}$, $R^{11}$ and/or $R^{12}$ together in each case may furthermore be ethylene or propylene, and its physiologically tolerated salts which comprises reacting

a) an alcohol of the formula II

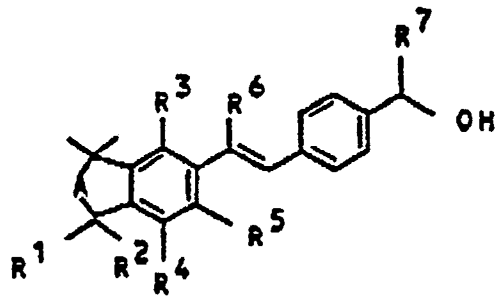

II

where A, $R^1$, $R^2$, $R^3$, $R^4$, $R^5$, $R^6$ and $R^7$ have the above meanings, with a phosphorylating reagent of the formula III

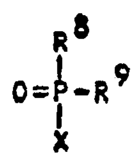

III

where $R^8$ and $R^9$ have the above meanings and X is halogen or an imidazole, N,N-dicyclohexylisourea, trichloroacetimidate, p-toluenesulfonyloxy, p-nitrophenyloxy or mesityloxy radical or a group $-O-P(O)R^8R^9$, or

b) an alcohol of the formula II under oxidative conditions with a phosphorylating reagent of the formula

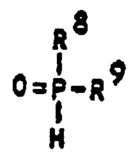

V,

where $R^8$ and $R^9$ have the above meanings,

and, if desired, converting the resulting compound to one of its physiologically tolerated salts.

2. A process as claimed in claim 1, wherein the compound prepared has the formula I set forth in claim 1 where A is a methylene or ethylene radical which is unsubstituted or substituted by methyl, $R^1$ and $R^2$ have the above meanings, $R^3$, $R^4$ and $R^5$ are each hydrogen, $R^6$ is hydrogen or methyl, $R^7$ is hydrogen and $R^8$ and $R^9$ are each hydroxyl or $C_1$-$C_4$-alkoxy.